# FASCICULE DE BREVET EUROPEEN

(11) **EP 1 087 997 B1**
(45) Date de publication et mention de la délivrance du brevet: **14.05.2008**
(21) Numéro de dépôt: 99925118.4
(22) Date de dépôt: 17.06.1999
(51) Int. Cl.: C07K 16/18, A61K 39/395, C12N 5/20, C12N 15/06, G01N 33/577, G01N 33/68

(54) **ANTICORPS MONOCLONAUX DIRIGES CONTRE LA PROTEINE G3BP, ET LEURS UTILISATIONS**
MONOKLONALE ANTIKÖRPER GEGEN DAS PROTEIN G3BP UND DEREN VERWENDUNGEN
MONOCLONAL ANTIBODIES DIRECTED AGAINST THE G3BP PROTEIN, AND USES

(30) Priorité: 17.06.1998 FR 9807617
(43) Date de publication de la demande: 04.04.2001
(73) Titulaire: Aventis Pharma S.A., 92160 Antony (FR)
(72) Inventeur: PARKER, Fabienne, F-92160 Antony (FR); KENIGSBERG, Mireille, F-91230 Montgeron (FR); DUCHESNE, Marc, F-94370 Sucy-en-Brie (FR); BARLAT, Isabelle, F-94510 La-Queu-en-Brie (FR)
(86) Numéro de dépôt international: PCT/FR1999/001453
(87) Numéro de publication internationale: WO 1999/065947

(56) Documents cités:
- WO-A-96/16169
- F. PARKER ET AL.: "A Ras-GTPase-activating protein SH3-domain-binding protein." MOLECULAR AND CELLULAR BIOLOGY, vol. 16, no. 6, juin 1996 (1996-06), pages 2561-2569, XP002092186 Washington, DC, états-unis cité dans la demande
- M. DUCHESNE ET AL.: "Identification of the SH3 domain of GAP as an essential sequence for Ras_GAP-mediated signaling." SCIENCE, vol. 259, no. 5094, 22 janvier 1993 (1993-01-22), pages 525-528, XP002092187 Washington, DC, états-unis

## Description

La présente invention concerne des anticorps monoclonaux dirigés contre la protéine G3BP ainsi que les lignées cellulaires les produisant. L'invention concerne également l'utilisation de ces anticorps ou de leurs dérivés pour l'obtention de médicaments et de réactifs de diagnostique.

Les produits des gènes ras, généralement désignés protéines p21 Ras, jouent un rôle clé dans le contrôle de la division cellulaire chez tous les organismes eucaryotes où ils ont été recherchés. Certaines modifications spécifiques de ces protéines leur font perdre leur contrôle normal et les conduisent à devenir oncogéniques. Ainsi, un grand nombre de tumeurs humaines ont été associées à la présence de gènes ras modifiés. De même, une surexpression des protéines p21 Ras peut conduire à un dérèglement de la prolifération cellulaire. Globalement, les protéines p21Ras sont impliquées dans 30% des cancers humains.

La compréhension du rôle exact de ces protéines p21 Ras constitue donc un des objectifs cibles de la recherche dans le domaine de l'oncologie.

Le modèle dont on dispose actuellement pour expliquer le fonctionnement des protéines p21 Ras repose sur des analogies qu'elles partagent avec les protéines G de transduction. Il existe dans les cellules un équilibre entre les protéines p21 actives, liées à du GTP et les formes inactives, ayant fixé du GDP. Dans une cellule quiescente, où les protéines Ras ne sont pas sollicitées, la majorité d'entre elles sont sous forme GDP. Lorsque la cellule est stimulée, le facteur d'échange des nucléotides, GEF, devient plus actif et facilite l'évacuation du GDP et son remplacement par du GTP. La protéine adopte alors une conformation active qui lui permet de reconnaître et de stimuler son effecteur, la protéine GAP "GTPase activating protein". Le complexe Ras-GTP-GAP interagit vraisemblablement à son tour avec une ou d'autres protéine(s) permettant ainsi la transmission du signal qui entraîne une réponse biologique de la cellule. L'association de Ras-GTP avec GAP déclenche simultanément l'hydrolyse du GTP et le retour de la protéine Ras vers la forme inactive, GDP.

Dans le cas des protéines p21Ras oncogènes, la mutation qu'elles portent empêche le retour à l'état inactif. L'équilibre est dans ce dernier cas, déplacé vers la forme active de Ras.

Cet équilibre complexe entre les formes active et inactive des p21Ras est contrôlé à la fois par des facteurs inhérents aux propriétés biochimiques des protéines Ras (affinité relative pour le GDP et le GTP, vitesse d'échange des nucléotides ...) et des facteurs externes modulant leur activité comme notamment la protéine GAP.

La protéine GAP est une protéine cytosolique présente chez tous les organismes eucaryotes qui possède donc la faculté d'accélérer fortement l'hydrolyse du GTP, lié à la protéine p21 normale ( Trahey et Mc Cormick 1987). Elle possède deux domaines assurant des fonctions distinctes. Son extrémité carboxy-terminale porte l'activité catalytique qui fixe les protéines Ras et augmente leur activité GTPase. Sur son autre extrémité, en aval de la partie amino-terminale, se trouve une juxtaposition de domaines SH2 et SH3 qui sont susceptibles de participer à des interactions avec d'autres protéines.

La demanderesse a déjà identifié une protéine supplémentaire dans la cascade de la signalisation Ras-dépendante. Ainsi, la demande WO96/16169 décrit l'identification, le clonage, le séquençage et la caractérisation d'une protéine capable de lier le domaine SH3 de GAP, désignée G3BP ("GAP-SH3 Binding Protein"). Cette demande montre en particulier que G3BP est un effecteur de GAP, c'est-à-dire un partenaire intervenant en aval dans la voie de signalisation Ras-dépendante. Ainsi, l'article de Parker et al (Mol. Cell. Biol. 16 (1996) 2561) décrit la séquence de cette protéine, sa capacité à lier GAP, etc. Cette protéine constitue une nouvelle cible majeure dans la mise au point d'approches thérapeutiques anti-cancéreuses. La demande WO96116169 indique la possibilité de produire des anticorps dirigés contre la proteine G3BP mais elle ne décrit pas d'anticorps capables d'induire l'apoptose dans des cellules qui surexpriment la proteine G3BP.

La protéine G3BP est une protéine cytosolique de 68 kDa exprimée de façon ubiquitaire. La séquence de la protéine G3BP et du gène correspondant sont présentées dans les SEQ ID n°1 et SEQ ID n° 2. Cette protéine de 466 acides aminés appartient à la famille des hnRNP (heterogeneous nuclear RiboNucleoproteins) et contient plusieurs domaines caractéristiques des protéines qui se lient à l'ARN :
- un domaine RRM (aa 342-385) avec les domaines RNP2 (aa 342-347) et RNP1 (aa 378 à 385)
- un domaine riche en acides aminés Arginine , Glycine (aa 429 à 461)
- un domaine auxiliaire acide (aa 144 à 221)

La demanderesse s'est maintenant intéressée au mécanisme d'action de la protéine G3BP. Dans cette optique, la demanderesse a obtenu différents anticorps dirigés contre différent domaines de la protéine G3BP afin d'antagoniser l'activité de G3BP ou de constituer des effecteurs de G3BP.

La présente invention repose d'une part sur la mise en évidence d'une surexpression de la protéine G3BP dans les cellules tumorales et dans les tumeurs humaines (adenocarcinome du colon, et tumeur du sein) et d'autre part sur la découverte surprenante que certains anticorps dirigés contre la protéine G3BP sont capables d'induire l'apoptose dans les cellules tumorales humaines. De manière inattendue, il a été constaté que ces anticorps induisent l'apoptose dans les cellules tumorales dans lesquelles la protéine G3BP est surexprimée mais se révèlent non toxiques dans les cellules humaines normales, où G3BP est faiblement exprimée.

Un premier objet de l'invention concerne des anticorps monoclonaux dirigés contre la protéine G3BP et capables d'induire l'apoptose dans différents types de cellules tumorales.

Plus particulièrement, l'invention a pour objet des anticorps monoclonaux capables de reconnaître un épitope situé dans la partie N-terminale de la protéine G3BP. De manière avantageuse, il s'agit d'un épitope compris entre les acides aminés situés aux positions 22 à 55 de la protéine G3BP, et plus préférentiellement encore d'un épitope constitué par les acides aminés situés aux positions 22-34.

Dans un mode particulièrement avantageux, la présente invention concerne des anticorps désignés par l'appellation Mab 1F1 et sécrétés par la lignée d'hybridome G3B 1F1 1D1 déposée le 9 juin 1998 auprès de la C.N.C.M. sous le numéro I-2038.

L'invention englobe également les anticorps dérivés des anticorps monoclonaux définis ci-avant. Au sens de la présente invention, on entend par anticorps dérivés toute molécule qui comprend l'idiotype des anticorps monoclonaux selon l'invention et notamment les anticorps chimériques, les anticorps simple chaîne et les fragments Fab. De tels anticorps chimériques peuvent être obtenus selon les techniques décrites par Morrison et al., J. Bacteriol. 159 : 870 (1984) ; Neberger et al., Nature 312:604-608 (1984) ; Takeda et al., Nature 314:452-454 (1985). Les fragments Fab qui contiennent l'idiotype des anticorps selon l'invention peuvent être générés par toute technique connue de l'homme du métier. Par exemple de tels fragments comportent de manière non limitative : le fragment F(ab')₂ , fragment qui peut être produit par digestion par la pepsine de l'anticorps ; les fragments Fab' qui peuvent être obtenus par réduction des ponts disulfure du fragment F(ab)₂ , et les fragments Fab qui peuvent être produits par traitement de l'anticorps avec la papaïne et un agent réducteur.

L'invention a également pour objet des anticorps simple chaîne ScFv dérivés des anticorps monoclonaux définis ci-avant. De tel anticorps simple chaîne peuvent être obtenus selon les techniques décrites dans les brevet US 4,946,778, US 5,132,405 et US 5,476,786.

L'invention concerne également une séquence d'acides nucléiques comprenant un gène codant pour un anticorps simple chaîne dérivé des anticorps monoclonaux décrits ci-avant. L'obtention de telles séquences et leur utilisation pour l'expression in vivo d'anticorps a été décrite dans la demande WO 94/29446 incorporée par référence à la présente.

L'invention a également pour objet des vecteurs viraux ou plasmidiques contenant une séquence d'acide nucléique codant pour un anticorps simple chaîne dérivé des anticorps monoclonaux décrits ci-avant. Plus particulièrement, lés vecteurs de l'invention sont d'origine virale, tels que les rétrovirus, les adénovirus, les virus adéno-associés, le virus de l'herpès, le virus de la vaccine, le virus HSV, etc.

L'invention concerne également l'utilisation de séquences d'acides nucléiques codant pour ces ScFv ou de vecteurs les incluant pour la préparation de compositions pharmaceutiques destinées au traitement chirurgical ou thérapeutique du corps humain ou animal. Elle concerne aussi toute composition pharmaceutique comprenant un vecteurs, notamment viral, ou une séquence d'acides nucléiques tels que définis ci-avant.

L'invention a également pour objet les lignées d'hybridomes susceptibles de sécréter les anticorps monoclonaux selon l'invention.

L'invention a plus particulièrement pour objet la lignée d'hybridome G3B 1F1 1D1 sécrétant l'anticorps Mab 1F1 déposée le 9 juin 1998 auprès de la Collection Nationale des Cultures de Microorganismes (C.N.C.M.) sous le numéro 1-2038.

L'invention concerne également un procédé de production d'anticorps monoclonaux capables d'induire l'apoptose dans différentes lignées tumorales comprenant (1) la fusion de cellules spléniques d'un animal immunisé à l'aide de la protéine G3BP ou d'un fragment comprenant au moins une partie du domaine N-terminal (aa 1-144), avec des cellules myelomateuses dans des conditions permettant la formation d'hybridomes ; (2), la détection et l'isolement de ceux desdits hybridomes qui sécrètent des anticorps monoclonaux capables d'induire l'apoptose dans différentes lignées tumorales.

L'invention concerne également l'utilisation des anticorps définis ci-avant pour l'obtention de médicaments. L'invention concerne plus particulièrement l'utilisation desdits anticorps pour l'obtention d'un médicament destinés au traitement ou à la prévention des désordres hyperprolifératifs.

L'invention a également pour objet des compositions pharmaceutiques comprenant une quantité thérapeutiquement efficace des anticorps selon l'invention, éventuellement mélangés à un support pharmaceutiquement acceptable, ladite quantité étant thérapeutiquement efficace pour induire l'apopotose dans des cellules tumorales.

Les anticorps selon l'invention peuvent également être utilisés comme réactif de diagnostic pour identifier ou doser la protéine G3BP. En effet, il a été constaté que la protéine G3BP est surexprimée dans les cellules tumorales et que cette surexpression constitue un marqueur dans les phénomènes de prolifération cellulaire. Ces anticorps sont donc particulièrement utiles pour diagnostiquer des maladies hyperprolifératives entraînant une surexpression de la protéine G3BP.

L'invention a donc également pour objet l'utilisation des anticorps monoclonaux définis ci-avant comme réactif de diagnostique ainsi qu'un kit de diagnostic immunologique comprenant lesdits anticorps monoclonaux.

Les anticorps peuvent être couplés à des marqueurs chromogènes, fluorescents, biotinylés, radioactifs ou autres ... Les anticorps peuvent être utilisés dans des méthodes d'immunomarquage en immunohistochimie, en cytométrie de flux, pour des dosages radioimmunologiques (RIA) ou immunoenzymatiques (ELISA) et dans tout type de kits de diagnostic connu.

Les anticorps selon l'invention ont également permis de réaliser une étude fonctionnelle de la protéine G3BP. En particulier, cette étude fonctionnelle a conduit à l'identification de nouveaux peptides dérivés de la protéine G3BP et qui présentent une activité apoptotique. A cet égard, l'invention a également pour objet de nouveaux peptides dérivés de la protéine G3BP et capable d'induire l'apoptose. De préférence, les peptides selon l'invention comprennent le fragment N-terminal, et de préférence encore ces polypeptides comprennent au moins le domaine correspondant aux 14 premiers acides aminés de la protéine G3BP.

### LEGENDES DES FIGURES

Figure 1 : Identification par Western Blot des différents domaines de G3BP reconnus par l'anticorps Mab 1F1
Figure 2 a et 2 b : Comparaison du niveau d'expression de la protéine G3BP dans différentes lignées cellulaires.
Figure 3 : Effet de la microinjection d'anticorps Mab 1F1 dans les cellules HCT116.
Figure 4 : Comparaison de séquences entre les protéines humaines G3BP et G3BP2. La comparaison des séquences N-terminales fait apparaître plusieurs différences mises en évidence par les trémas.

### MATERIELS ET METHODES

### Lignées cellulaires utilisées

HCT1 16: cellules épithéliales issues de carcinome de colon humain présentant un gène *Ki-ras* muté et une mutation du gène suppresseur de tumeur DCC. Milieu de culture requis : DMEM, 10% Serum de veau foetal, 1% Penicilline/ Streptomycine, 1 % Glutamine.

Hke-3 et HK2-6 : cellules HCT116 modifiées par délétion du gène *Ki-ras* et qui, de ce fait ont perdu leur pouvoir tumorigène. (ref: Shirasawa,..Sasazuki. (1993) Science 260, 85-88). Milieu de culture requis : DMEM, 10% Serum de veau foetal, 400µg/ml Geneticine, 1% Penicilline/ Streptomycine, 1% Glutamine.

H460 : cellules épithéliales issues de carcinome pulmonaire non à petites cellules humain présentant un gène *Ki-ras* muté. Milieu de culture requis : DMEM, 10% Sérum de veau foetal, 1% Penicilline/ Streptomycine, 1% Glutamine.

H1299 : provenance ATCC. Cellules épithéliales issues de carcinome pulmonaire humain présentant un gène *N-ras* muté et une délétion du gène p53. Milieu de culture requis : DMEM, 10% Serum de veau foetal, 1% Penicilline/ Streptomycine, 1% Glutamine.

HeLa : cellules épithéliales issues de carcinome humain du col de l'utérus ne présentant pas de mutation des gènes *ras.* Milieu de culture requis : DMEM, 10% Sérum de veau foetal, 1% Pénicilline/ Streptomycine, 1% Glutamine.

HT-29 : provenance ATCC. Cellules épithéliales issues de carcinome de colon humain présentant une mutation du gène p53 mais pas de mutations des gènes *ras.* Milieu de culture requis : DMEM, 10% Sérum de veau foetal, 1% Pénicilline/ Streptomycine, 1% Glutamine.

DLD-1 : provenance ATCC. Cellules épithéliales issues de carcinome de colon humain présentant une mutation du gène *Ki-ras* et du gène p53. Milieu de culture requis : DMEM, 10% Sérum de veau foetal, 1% Pénicilline/ Streptomycine, 1% Glutamine.

T47 : provenance ATCC. Cellules isolées à partir d'effusion pleurale d'une patiente atteinte de carcinome mammaire. Milieu de culture requis : RPMI, 10% sérum de veau foetal, 1% Pénicilline/ Streptomycine, 1 % Glutamine.

BT20 : Cellules issues de carcinome mammaire humain. Milieu de culture requis : RPMI, 10% sérum de veau foetal, 1% Pénicilline/ Streptomycine, 1% Glutamine.

MCF-7 : Cellules issues de carcinome mammaire humain. Milieu de culture requis : RPMI, 10% sérum de veau foetal, 1% Pénicilline/ Streptomycine, 1% Glutamine.

T24 : provenance ATCC. Cellules issues de carcinome humain de la vessie, présentant une mutation du gène *Ha-ras.* Milieu de culture requis : RPMI, 10% sérum de veau foetal, 1% Pénicilline/ Streptomycine, 1 % Glutamine.

NHDF : provenance Boerhinger Ingelheim. Normal Human Dermal Fibroblasts (adult) en culture primaire ne présentant pas de gènes mutés. Milieu requis du fournisseur.

### EXEMPLES

### Exemple 1 : Obtention des anticorps monoclonaux

### 1.1 - Production de la protéine G3BP

Les cultures de cellules SF9 ont été réalisées selon le protocole décrit dans Parker et al (Mol. Cell. Biol. 16 (1996) 2561). Les cellules sont lysées en présence de tampon HNTG (50 mM d'Hépès, pH 7,5, 150 mM de NaCl, 1 % de Triton X 100, 10 % de Glycérol, 1mM de %gCl2, 1mM d'EGTA; en présence d'inhibiteurs de phosphatases (1mM Na3VO4, 10mM de Na4P2O7, 10 mM de NaF) et d'inhibiteurs de protéases (1µg/ml de leupeptine, 1 µg/ml d'inhibiteur de trypsine, 1 µg/ml de pepstatine A, 2 µg/ml d'aprotinine, 10 µg/ml de benzamidine, 1 mM de phénylméthanesulfonyle fluorure, 1 µg/ml d'antipaïne, 1 µg/ml de chymostatine). Le lysat est centrifugé (15,000 g, 15 min.) et dilué 5 fois dans du tampon de lavage HG (50 mM Hepes pH 7.5, glycérol 10 %, 1 mM EGTA, en présence d'inhibiteurs de phosphatases et d'inhibiteurs de protéases). Le lysat est ensuite incubé 12 heures en présence du gel Heparin-Sepharose Fast Flow gel (Pharmacia LKB) à raison de 15 mg de protéines par millilitre de gel, équilibré dans le même tampon (50 mM Hepes, 0.030 M NaCl, 0.2 % Triton X100, glycérol 10 %, MgC12 1 mM, 1 mM EGTA). Le complexe est transféré dans une colonne K26 Pharmacia. La colonne est lavée avec 10 volumes de tampon d'équilibration (50 ml/heure), puis les protéines sont éluées en tampon NaCl 100 mM puis en tampon NaCl 600 mM. Les fractions contenant l'activité de liaison GAP-SH3 sont rassemblées, diluées 10 fois en tampon HG et chargées sur une colonne d'agarose-polyribouridylic acid AGPOLY(U), (colonne de type 6, 19 cm² x 1 cm, Pharmacia) à raison de 1.3 mg de protéines par millilitre de gel. La colonne est pré-équilibrée dans le même tampon avec un débit de 5 ml / cm² h⁻¹. Après lavage en tampon HNG (60 mM NaCl), les protéines contaminantes sont éluées avec 229 ml d'un tampon en gradient linéaire de 0.06 à 0.32 M NaCl, et la protéine G3BP est éluée en présence de NaCl 0.7 M. A l'issue de la chromatographie POLY(U), les fractions contenant la protéine G3BP sont rassemblées, diluée 12 fois en présence de tampon phosphate pH 7.5 + inhibiteurs de protéases) et chargées sur une colonne MonoS HR5/5 1 ml (Pharmacia), pré-équilibrée dans le même tampon contenant 60 mM NaCl, à un débit de 30 ml h⁻¹. Après rinçage de la colonne, les protéines sont éluées avec 40 ml d'un tampon en gradient linéaire de 0.06 à 1 M NaCl. La protéine G3BP est éluée entre 0.15 M et 0.2 M NaCl.

### 1.2 - Protocole d'immunisation des souris

Des souris femelles BALB/c agées de 6 semaines ont reçu une injection intrapéritonéale de 25 µg de G3BP au jour J0 de l'immunisation. Une deuxième injection intrapéritonéale a été faite au jour J14 de l'immunisation puis une troisième et quatrième injections aux jours J45 et J80. Une cinquième injection (10 µg de protéine G3BP) est a été faite par voie intra-veineuse au jour J165 et les splénocytes ont été extraits trois jours plus tard et fusionnés. Les splénocytes ont été fusionnés avec des cellules murines de myélome SP2/O-Ag14 d'une part et X63Ag8/653 d'autre part. Le rapport splénocytes/cellules de myélome est de 5 : 1. L'hybridation lymphocytaire est faite en présence de polyéthylèneglycol PEG pm 1500 (concentration finale 40 %). Il a été préparé 16 plaques de 96 puits en présence de 10.000 macrophages péritonéaux de souris Balb/C par puits. Les quantités de splénocytes utilisées varient entre 2,5 10⁴ et 10⁵ cellules par puits. Le milieu utilisé contient RPMI 1640 (Biowhittaker et Gibco), 2 mM glutamine, 1 mM sodium pyruvate, HAT, hypoxantine 100 mM, aminoptérine 0.4 µM, thymidine 16 µM, 220 % sérum de veau foetal décomplémenté, pénicilline 100 U/ml, streptomycine 100 µg/ml.

Les premiers clones apparaissent trois jours après la fusion, le milieu est changé sept jours après la fusion, enfin 10 jours après la fusion, 5000 macrophages sont ajoutés par puits. Les puits dans lesquels un seul hybridome a poussé sont identifiés et les surnageants sont prélevés pour le criblage.

Les surnageants d'hybridome issus de la fusion sont criblés par Western Blot. Des extraits cellulaires (50 µg) de cellules ER22 et de cellules HCT116, la protéine G3BP recombinante ainsi qu'une protéine contrôle SAM68 ont été soumis à une électrophorèse sur gel de polyacrylamide à 7,5 % de dodecyl sulfate de sodium (SDS-PAGE) puis transférés sur une membrane de difluorure de polyvinylidène (PVDF Millipore Corpo.). La liaison non spécifique est bloquée par 2 % de lait écrémé dans du tampon ECL (20 mM Tris, pH 7.4, 150 mM NaCl, 0.05 % de Tween 20) pendant 2 heures, à température ambiante.

Les membranes sont ensuite incubées avec les différents surnageants dilués au 1/50 ème dans le tampon bloquant pendant une nuit à 4 °C. Après lavage avec le tampon ECL - 0.05 % Tween 20, les protéines liées sont détectées par incubation avec un anticorps anti-souris conjugué à une peroxidase et du réactif chemiluminescent ECL (NEN Life Science Product).

Trois hybridomes ont été retenus à l'issue de ce criblage. Il s'agit des hybridomes G3B 1E1, G3B 1F1 1D1 et G3B11H7.

### Exemple 2 - Identification de l'épitope reconnu par l'anticorps Mab 1F1.

Différents fragments de la protéine G3BP ont été produits dans un système d'expression en cellule d'insectes (SF9) infectées par un baculovirus recombinant selon la technique décrite dans Parker et al (Mol. Cell. Biol. 16 (1996) 2561). Il s'agit des fragments « 1 » (aa 72 - 350), « 2 » (aa 72 - 235), « 3 » (aa 144 - 341), « 4 » (aa 236 - 350), et « 5 » (aa 299 - 466). Des expériences de Western blot ont été réalisées à partir de ces fragments produits en système baculovirus .

### 2.1 - Détection des protéines par Western Blot

Après élimination du milieu de culture, les cellules en prolifération sont lavées deux fois en tampon phosphate à 4 °C. La lyse est effectuée directement sur les boites de culture en présence de 1 ml de tampon HNTG (HEPES pH 7,5 50 mM, NaCl 150 mM, Triton X-100 1 %, glycerol 10 %, MgCl2 1mM, 1 mM + inhibiteurs de phosphatases (Na₃VO₄ 1 mM, Na₄ P₂O₇ 10 mM, NaF 10 mM) + inhibiteurs de protéases (leupeptine 1 µg/ml, inhibiteur de trypsine 1 µg/ml, pepstatine A 1 µg/ml, benzamidine 10 µg/ml, phenylmethylsulfonyl fluoride 1mM, antipaine 1 µg/ml chymostatin 1 µg/ml). Les protéines sont séparées sur gel d'électrophorèse acrylamide Sodium Dodecyl-Sulfate (SDS)-Polyacrylamide (PAGE) de 7.5 % (16 heures, 60 volts) les protéines sont ensuite transférées sur membrane de polyvinylidene difluoride (PVDF) Millipore Corp.) par une méthode de transfert semi-liquide. Le transfert est réalisé à 4 °C, pendant 3 heures, sous tension de 60 volts, dans un tampon de transfert Tris Base 25 mM, Glycine 192 , SDS 0.15 % méthanol 20 %. La membrane est rincée en PBS puis saturée dans du PBS additionné de 0.05% de tween 20 et de 2 % de lait écrémé pendant 2 heures à température ambiante. La membrane est incubée pendant 12 h à 4 °C, dans un tampon ECL (20 mM TRIS, pH 7.4, 150 mM NaCl) + 0.05 % Tween 20 + 2 % lait écrémé, contenant le premier anticorps. L'anticorps MAB 1F1 est dilué au 1/10000 ème. La membrane est ensuite rincée 4 ou 5 fois pendant 10 minutes, à température ambiante dans un tampon ECL + 0.05 % Tween 20. Le second anticorps (anticorps anti-souris) est ensuite ajouté en présence de ECL + 0.05 % Tween 20 pendant 45 minutes à température ambiante. Les anticorps sont couplés à la peroxydase qui permet la révélation des protéines par la technique de l'Enhanced ChemiLuminescence (ECL). La membrane est rincée 4 ou 5 fois dans du tampon PBS + 0.05 % Tween 20, incubée 1 minute dans le réactif ECL. La révélation est effectuée selon les recommandations du fournisseur (NEN Life Science Product).

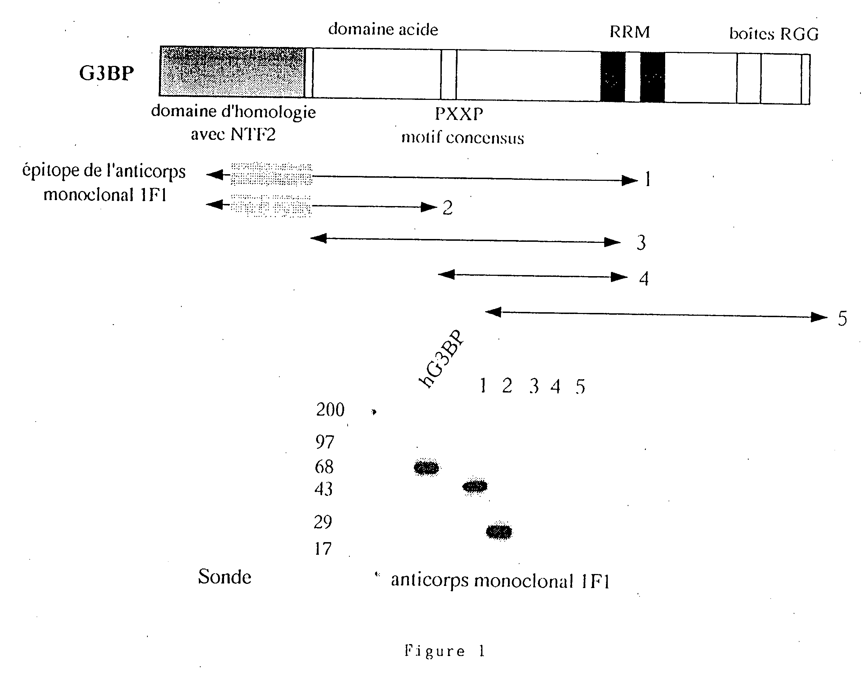

Les résultats sont présentés sur la figure 1 et montrent que l'anticorps Mab 1F1 permet la détection des fragments « 1» (aa 72 - 350) et « 2 » (aa 72 - 235). Le fragment « 3 » (aa 144 - 341) central comportant une zone commune au fragment « 2 » n'est pas reconnu. Les fragments « 4 » (aa 236 - 350) et « 5 » (aa 299 - 466) ne sont pas non plus reconnus.

Ces résultats montrent que l'épitope reconnu par l'anticorps Mab 1F1 est localisé dans la région N- terminale de la protéine de G3BP, dans la zone correspondant aux 144 premiers acides aminés et plus particulièrement dans la zone comportant les 100 premiers amino acides situés en amont du domaine acide (aa 144 - 221) de la protéine G3BP.

### Exemple 3 : niveau d'expression de la protéine G3BP dans différentes lignées cellulaires.

Le niveau d'expression de la protéine G3BP a été étudié dans des tumeurs colorectales humaines et dans différentes lignées tumorales.

### 3.1 - Niveau d'expression de G3BP dans diférentes lignées cellulaires humaines.

Pour cette expérience, les cellules en culture sont récoltées par scrapping et centrifugées 5 mn à 2000 t/mn pour les collecter en culot. Elles sont alors lysées dans un tampon Hepès 10 mM pH7.2, KCl 140 mM, MgCl2 5 mM, NP40 0.2% et inhibiteurs de protéases pendant 45 mn à 4 °C. Les lysats sont centrifugés à 14 000t/mn pendant 10 mn et les surnageants sont prélevés. Un dosage de protéines est effectué et les surnageants sont dénaturés en tampon de Laemmli 4X et traités 10 mn à 95°C.

L'équivalent de 50 µg de protéines des différents lysats est déposé par puits d'un minigel SDS-PAGE Novex 10% d'acrylamide, 10 puits et 1.5mm d'épaisseur.

Après migration, électrotransfert sur membrane de PVDF, et passivation des membranes en tampon TTBS [TRIS 20 mM pH 7.5, NaCl 150 mM, Tween 20 0.1 %, azide de sodium 0.02 %] contenant 3 % de BSA, les échantillons sont soumis à un Western-blot :

Les membranes sont incubées pendant 2 heures avec l'anticorps Mab 1F1 dilué à 0.2µg/ml dans du tampon TTBS contenant 3% de BSA, elles sont ensuite rincées quatre fois avec du TTBS pendant 10 mn, puis incubées 1 heure avec un anticorps anti-souris marqué à la péroxidase et à nouveau rincées quatre fois avec du TTBS pendant 10 mn. Les membranes sont ensuite révélées avec le kit ECL (chimie luminescence) NEN-Dupont sur film ECL Amersham.

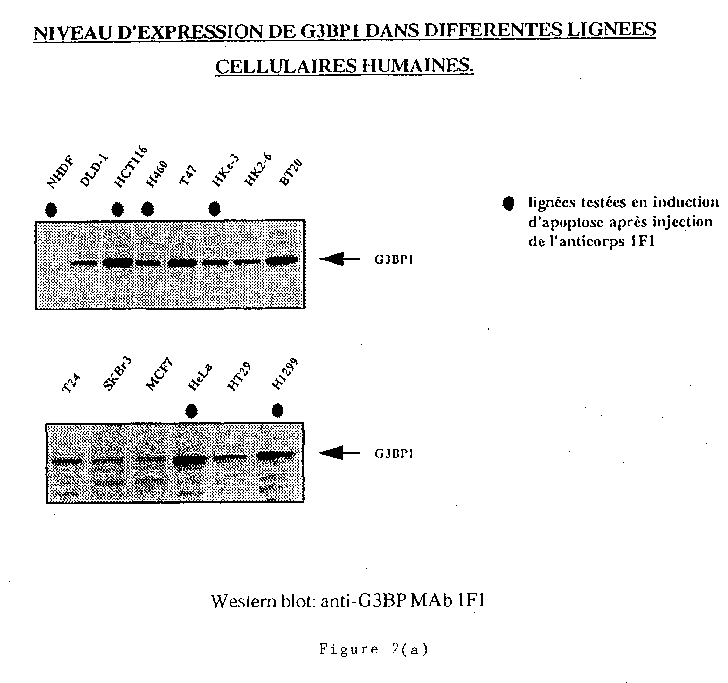

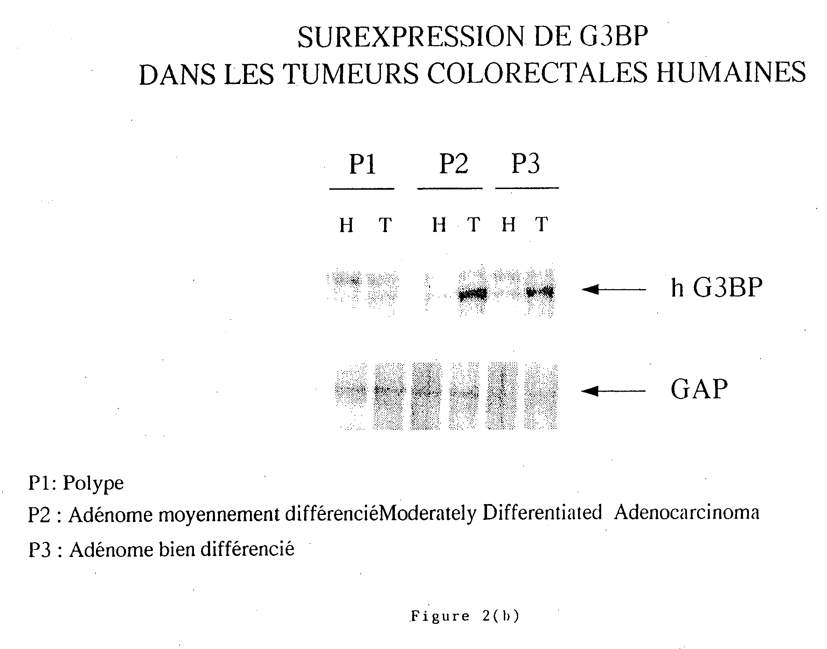

Les résultats sont présentés à la figure 2 a. Le film obtenu montre la surexpression de la protéine G3BP dans toutes les lignées tumorales testées, par rapport à la lignée de fibroblastes normaux NHDF. Le niveau de cette surexpression est variable d'une lignée à l'autre mais n'est pas dépendant de l'état d'activation des gènes *ras* dans ces lignées ; les lignées cellulaires présentant un gène *Ki-ras* muté comme les HCT116, H460 ou DLD-1, expriment plus ou moins fortement la protéine G3BP et les lignées cellulaires ne présentant pas de mutation des gènes *ras* comme les HeLa ou HT29, ont un niveau d'expression de G3BP tout aussi fort et variable.

On peut néanmoins constater que les cellules HKe-3 et HK2-6 dans lesquelles le gène *Ki-ras* a été délété, voient le niveau d'expression de G3BP diminuer par rapport aux cellules dont elles sont issues, les HCT116, sans pour autant qu'il y ait extinction de cette expression.

### Niveau d'expression de G3BP dans les biopsies humaines.

Les prélèvements de tissus sains ou tumoraux ont été fixés sur lame. La solubilisation des échantillons est effectuée par un dépôt de 40 µl de tampon HNTG sur chaque lame au niveau du tissu.Les extraits sont centrifugés à 14000t/min pendant 10 minutes et les surnageants sont prélevés. Les protéines sont dosées et dénaturées par du tampon de Laemmli 4X par un traitement à la chaleur 95 °C pendant 10 minutes.

Des échantillons de 20 à 30 µg de protéines ont été soumis à une électrophorèse sur gel de polyacrylamide à 7,5 % de dodecyl sulfate de sodium (SDS-PAGE) puis transférés sur une membrane de difluorure de polyvinylidène (PVDF Millipore Corpo.). La liaison non spécifique est bloquée par 2 % de lait écrémé dans du tampon ECL (20 mM Tris, pH 7.4, 150 mM NaCl, 0.05 % de Tween 20) pendant 2 heures, à température ambiante.

Les membranes sont ensuite incubées avec l'anticorps Mab 1F1 dilué à 0.2 µg/ml dans le tampon bloquant pendant une nuit à 4°C. Après lavage avec le tampon ECL - 0.05 % Tween 20, les protéines liées sont détectées par incubation avec un anticorps anti-souris conjugué à une peroxidase et du réactif chemiluminescent ECL (NEN Life Science Product)

Les résultats présentés sur la figure 2 b montrent que la protéine G3BP est surexprimée dans les adénomes moyennement et bien différenciés.

### Exemple 4 : Effet de la microinjection de l'anticorps Mab 1F1 sur la viabilité des cellules tumorales humaines.

Cet exemple illustre la propriété des anticorps selon l'invention à induire l'apoptose dans différents types de cellules tumorales.

L'anticorps monoclonal Mab 1F1 a été injecté selon le protocole décrit ci-après. La solution à injecter est dialysée contre du PBS, passée sur filtre 0.45µm UltraFree Millipore et centrifugée (5 mn à 14000 rpm) avant d'être introduite dans le microcapillaire à l'aide d'une pointe effilée « microloader » (Roucaire). Le temps d'injection est réglé de 0,1 à 0,3 seconde et la pression d'injection entre 50 hPa et 150 hPa selon la taille des cellules. Environ 200 cellules ont été injectées par condition.

Les cellules HCT116, H460, H1299, HeLa ou HKe-3 ont été microinjectées par 10 mg/ml d'anticorps monoclonal Mab 1F1 puis filmées et enregistrées par vidéomicroscopie (à 2 images par minute) pendant 3 jours après injection. A la lecture du film, l'évolution des cellules injectées est observable (divisions, morts cellulaires, changements morphologiques) et quantifiable (nombre de cellules se divisant ou mortes par heure). Seules les cellules qui se détachent du support après condensation et fragmentation (événements observables des cellules apoptotiques) sont comptées.

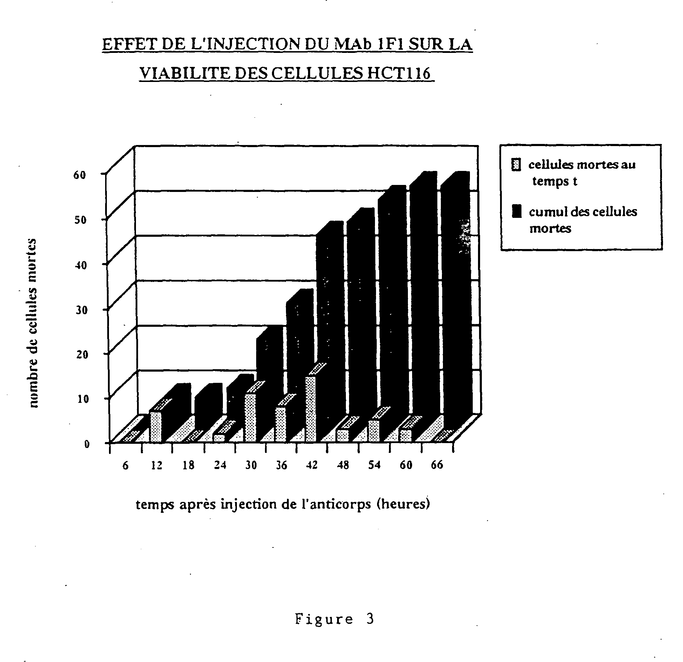

Les résultats obtenus avec l'injection de l'anticorps Mab 1F1 dans les cellules HCT 116 sont reportés dans la figure 3. On constate un pourcentage élevé de mort cellulaire induite à partir de douze heures après l'injection de l'anticorps Mab 1F1, avec un pic entre 24 et 48 heures.

Des doses différentes d'anticorps Mab 1F1 ont été testées dans les cellules HCT116. De 10 mg/ml à 3 mg/ml, l'effet d'induction de mort cellulaire est équivalent, c'est-à-dire que l'anticorps Mab 1F1 induit la mort cellulaire d'au moins 50% des cellules injectées. A 1 mg/ml d'anticorps, l'effet est fortement diminué.

Si les cellules HCT116 sont injectées par une immunoglobuline de souris à la concentration de 10 mg/ml comme contrôle, aucune mort n'est constatée et les cellules injectées continuent à se diviser normalement.

Cet effet toxique de l'anticorps Mab 1F1 dans les cellules HCT 116 a été confirmé par l'utilisation de la méthode « TUNEL » en fluorescence, qui permet de détecter spécifiquement les coupures dans l'ADN des cellules entrant en apoptose:

Les cellules sont ensemencées sur des lamelles de verre quadrillées (cellocates-Roucaire), et placées dans des plaques 4 puits. 2 jours après l'ensemencement, les cellules sont injectées sur ces lamelles par l'anticorps Mab 1F1 ou avec une immunoglobuline de souris, comme contrôle, à 10 mg/ml. Après injection, les cellules sont replacées à l'étuve pendant 40h. Elles sont alors fixées à la formaldéhyde 4% pendant 15 mn, perméabilisées 5 mn au triton X100 à 0.2% et rincées plusieurs fois avec du tampon phosphate. On procède alors au double marquage pendant 1h à 37°C avec :
- un anticorps anti-souris marqué au Texas Red dilué au 1/400, qui révélera les cellules injectées,
- kit Boerhinger « In situ cell death detection » qui marquera spécifiquement en vert, les coupures dans l'ADN des cellules apoptotiques.

Les lamelles sont ensuite montées sur lames de verre en milieu de montage (Vectashield-Biosys).

A l'observation en lumière fluorescente, à l'aide de filtres sélectionnant les différentes couleurs, les cellules injectées d'une part et les cellules en apoptose d'autre part, sont comptabilisées.

On obtient ainsi, dans 3 expériences indépendantes, entre 9 et 18% de cellules HCT116 mortes par apoptose au temps 40h. Dans les mêmes conditions, on ne constate la mort d'aucune cellule HCT116 injectée par l'immunoglobuline contrôle.

Les expériences d'enregistrement après microinjection de l'anticorps Mab 1F1, ont été reproduites avec les cellules H460, H1299, HeLa et HKe-3 avec des résultats similaires au graphe présenté dans la figure 3 montrant qu'au moins 50% des cellules injectées meurent de 12 à 60h après injection.

Les expériences de marquage « TUNEL » ont été réalisées avec les cellules HCT116 et H460 et ont donné des résultats équivalents (de 9 à 18% des cellules entrant en apoptose 40 heures après injection de l'anticorps).

### Exemple 5 : Effet de la microinjection de l'anticorps Mab 1F1 sur la viabilité et sur la prolifération des fibroblastes humains normaux NHDF

### 5.1 - Effet de la microinjection de l'anticorps Mab 1F1 sur la viabilité des fibroblastes humains normaux NHDF.

Les expériences d'enregistrement des cellules NHDF en prolifération microinjectées par l'anticorps Mab 1F1 montrent qu'aucune mort cellulaire n'est constatée dans les 60h après injection et que les cellules continuent à se diviser normalement.

### 5.2 - Effet de la microinjection de l'anticorps Mab 1F1 sur la prolifération des fibroblastes humains normaux NHDF.

Les cellules normales en culture ont besoin de l'apport en facteurs de croissance. Si elles sont privées de ces facteurs elles entrent dans une phase de « non-prolifération » dite de quiescence où elles ne répliquent plus leur ADN et ne peuvent donc plus incorporer la bromodéoxyuridine, un analogue de la thymidine ajouté dans le milieu de culture, que seules les cellules en prolifération peuvent incorporer dans leur ADN.

Lorsqu'on microinjecte dans des cellules NHDF quiescentes, l'anticorps Mab 1F1 à la concentration de 10 mg/ml, et que l'on remet ces cellules dans un milieu contenant des facteurs de croissance et de la bromodéoxyuridine (BrdU), on constate, après 24h de contact puis fixation et coloration à l'aide d'un anticorps anti-BrdU, une inhibition de 33% de la prolifération cellulaire.

Cette inhibition est calculée en rapportant le pourcentage de cellules injectées par l'anticorps Mab 1F1 et qui ont incorporé la BrdU, au pourcentage de cellules injectées par une immunoblobuline de souris et qui ont incorporé la BrdU (contrôle).

Un autre anticorps monoclonal anti-G3BP (11H7) a été injecté dans les cellules NHDF dans les mêmes conditions.

**Tableau 1 : effet de l'injection d'anticorps spécifiques de G3BP sur la croissance cellulaire de cellules NHDF**

| Anticorps injecté | % d'incorporation BrdU |
|---|---|
| Immunoglobuline de souris (IgG) | 44.7 |
| Mab 1F1 | 29.7 |
| 11H7 | 30.3 |

On constate que l'immunoglobuline n'empêche pas la prolifération cellulaire alors que l'anticorps 11H7 a un effet similaire à celui de l'anticorps Mab 1F1, à savoir qu'ils provoquent une inhibition de la prolifération de 32 à 33%.

On attribue cette inhibition au blocage de la protéine G3BP endogène par l'anticorps Mab 1F1 ou 11 H7. A cet égard il est intéressant de noter que cet arrêt de prolifération n'est que temporaire dans les cellules normales NHDF et n'entraine en aucun cas la mort des cellules. En effet, les cellules reprennent leur cycle normal lorsque l'anticorps est naturellement dégradé dans le cytoplasme (environ 40h après l'injection).

Les résultats présentés dans les exemples 4 et 5 montrent que l'anticorps Mab 1F1 induit l'apoptose dans au moins 50% des cellules tumorales humaines testées dans lesquelles G3BP est surexprimée mais se révèle non toxique lorsqu'il est injecté dans les cellules humaines normales, où G3BP est faiblement exprimée. Cet effet dans les cellules tumorales n'est apparemment pas lié à l'état d'activation des gènes *ras.* Par ailleurs, l'anticorps Mab 1F1, provoque un arrêt temporaire - ou un retard - de la prolifération des cellules normales NHDF, lorsqu'il est injecté dans les cellules quiescentes.

### Exemple 6 - Clonage et expression d'une séquence d'ADN codant pour un anticorps intracellulaire anti -G3BP à partir de l'hybridome G3B 1F1 1D1.

Cet exemple décrit le clonage et l'expression d'une séquence d'acides nucléiques codant pour un anticorps intracellulaire reproduisant les propriétés de l'anticorps monoclonal Mab 1F1.

### 6.1 - Préparation de la séquence d'ADN.

Une séquence d'ADN codant pour un anticorps intracellulaire (fragment ScFv) est préparée selon la technique décrite dans le brevet US 4,946,778. Cette séquence est ensuite placée sous le contrôle d'un promoteur fonctionnel dans les cellules mammifère.

Les ARN poly A sont isolés à partir d'une culture cellulaire de l'hybridome G3B 1F1 1D1, selon la technique décrite par Chirguin S.H. et al (Biochemistry 18, 5294 (1979)). Ces ARN sont utilisés pour une réaction de Réverse-Transcription à l'aide de primers murins formés d'hexanucléotides aléatoires. Les ADNc obtenus servent de matrice à deux réactions de PCR
- l'une destinée à amplifier le fragment variable de la chaine lourde (VH) avec des primers VH spécifiques,
- une deuxième PCR permettant d'obtenir le fragment VL , en utilisant un mélange de 10 primers dérivés des séquences murines.

Deux fragments de 340 bp et 325 bp sont ainsi obtenus et assemblés grâce à un linker qui permet un positionnement correct de l'ADNc de VH en 5' de celui de VL. PCR (Recombinant Phage Antibody System Mouse ScFv Module, Pharmacia, 27-9400-01).

La séquence d'acides nucléiques fusionnée VH-linker-VL est ensuite insérée dans un phagemide qui permet l'expression de l'anticorps intracellulaire. Cette expression permet facilement l'identification et la sélection des anticorps intracellulaires qui reconnaissent correctement l'antigène. Cette étape est réalisée en sélectionnant les anticorps capables de reconnaître G3BP en test ELISA (Recombinant Phage Antibody System Expression Module, Pharmacia, 27-9401-01).

### 6.2 - Evaluation fonctionnelle de l'anticorps intracellulaire modifié.

La séquence qui code pour l'anticorps intracellulaire modifié anti-G3BP est isolée du phagemide par restriction, puis insérée dans le vecteur SV2 (Schweighoffer et al., Science, 256, 825-827 (1992)) ou pCDNA3 ( InVitrogen, V790-20). Le plasmide ainsi obtenu est appelé pSV-ScFvG3BP ou pCDNA3ScFvG3BP. L'évaluation fonctionnelle peut être réalisée au moyen des tests décrits ci-dessous.
a) par microinjection dans les cellules mammifères non immortalisées NHDF et dans des lignées transformées (HCT116, H460, Hke-3, H1299, Hela) en examinant l'apoptose dans les lignées transformées suivant les techniques décrites précédemment et en mesurant l'impact de l'anticorps intracellulaire anticorps sur la viabilité cellulaire par comparaison avec l'activité de l'anticorps Mab 1F1.
b) par un test de néo-résistance sur les lignées transformées et les cellules normales. Le plasmide pCDNA3G3BP est transfecté dans les lignées transformées (HCT116, Hke-3, H460, H1299, Hela) ainsi que dans des cellules humaines non transformées. Trois jours après transfection, les cellules transformées sont sélectionnées en présence de généticine . Dans ce test, l'anticorps simple chaîne est comparé à des fragments de G3BP (dont les séquences d'acides nucléiques correspondantes sont incluses dans les mêmes vecteurs d'expression) qui inhibent la croissance des cellules tumorales.
c) par l'inhibition de la formation de foyers. La capacité de l'anticorps simple chaîne à inhiber la formation de foyers est réalisée selon la technique décrite dans WO94/29446. Pour les tests de diminution de la capacité tumorale, l'effet peut également être recherché dans le cas de transformation par d'autres oncogènes que Ras.
d) en recherchant l'impact de l'expression de ce ScFv sur l'activité de liaison à l'ARN et/ou la coupure endonucléolytique de l'ARN par G3BP1. En effet, l'apoptose induite par microinjection de l'anticorps monoclonal Mab 1F1 peut s'expliquer soit par la dissociation d'une interaction avec un partenaire protéique spécifique, soit par une modification de l'activité de liaison à l'ARN et/ou de clivage endoribonucléolytique. L'anticorps ScFv obtenu peut également être caractérisé par sa capacité à modifier ces interactions.

### Exemple 7 : Identification d'un autre domaine de reconnaissance de l'anticorps Mab 1F1 sur la protéine G3BP1. Comparaison de séquences entre les protéines humaines G3BP et G3BP2 (ou G3BPh).

La protéine G3BP fait partie d'une famille de protéines dans laquelle se trouvent les protéines G3BP2 courte (AF 051311, Genbank) qui présente 60.9% d'identité au niveau nucléotidique, et 58.9% au niveau protéique avec G3BP , et la protéine G3BP2 longue (AB 014560, Genbank). La protéine G2BP2, forme longue, présente une insertion de 99 nucléotides en 790 dans la séquence de G3BP2 (forme courte).

La comparaison des séquences protéiques entre les protéines G3BP et G3BP2 fait apparaître une conservation des principaux domaines de la protéine, à savoir :
1 - une région N-Terminale (présentant des homologies avec la protéine NTF2, Nuclear Transport Factor 2), cible de l'anticorps monoclonal Mab1F1,
2 - une région acide,
3 - une région riche en motifs PXXP impliqués dans les interactions protéines-protéines,
4 - une région comportant des domaines RRM de reconnaissance à l'ARN, et des motifs auxilliaires, des boites « RGG »(arginine, glycine, glycine).

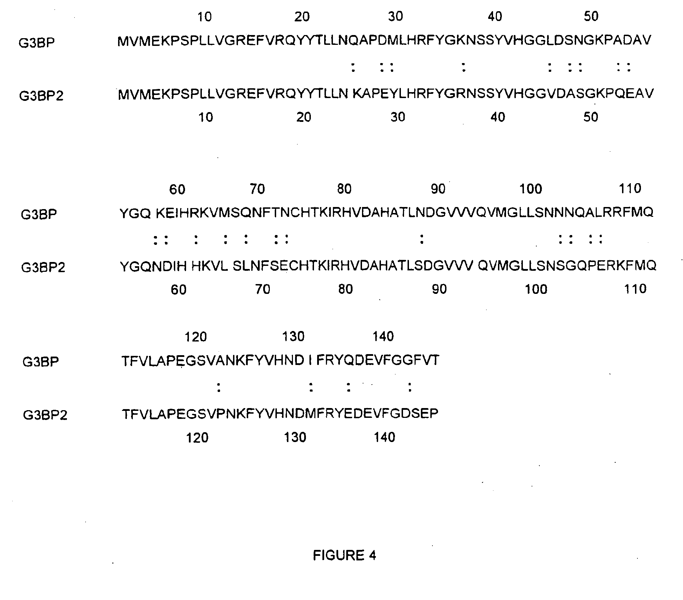

De façon remarquable, l'anticorps monoclonal Mab1F1 n'est pas capable de reconnaître la protéine recombinante G3BP2 (forme courte) dans des expériences de Western-blot. La comparaison des séquences N-terminale fait apparaître plusieurs différences mises en évidence par les trémas dans la figure 4.

L'utilisation de peptides recouvrants les différentes régions divergentes a permis de préciser le domaine de reconnaissance de l'anticorps sur la protéine G3BP.

Dans un premier temps, la comparaison des séquences des protéines décrite ci-dessus a permis de synthétiser différents peptides (A-F) :
Peptide A : LLNQAPDMLHRFY
acides aminés 22-34 de la protéine G3BP
Peptide B : LLNKAPEYLHRFY
acides aminés 22-34 de la protéine G3BP2
Peptide C : HGGLDSNGKPADAV
acides aminés 42-55 de la protéine G3BP
Peptide D : HGGVDASGKPQEAV
acides aminés 42-55 de la protéine G3BP2
Peptide E : LLSNNNQALRRFMQ
acides aminés 97-11 de la protéine G3BP
Peptide F : HNDIFRYQDEVFG
acides aminés 127-139 de la protéine G3BP

Ces peptides ont ensuite été testés :
(i) pour leur capacité à reconnaître l'anticorps Mab1F1 dans un test ELISA,
(ii) pour leur capacité à inhiber la reconnaissance de la protéine G3BP par l'anticorps Mab1F1 dans des expériences de Western-blot.

### (i) Test des peptides A-F dans un test ELISA

Les peptides en solution à 10mg/ml dans du tampon carbonate (0.1M, pH 9.6) ont été fixés par adsorption pendant une nuit à 4°C sur des plaques 96 puits. Après un lavage en PBS , les puits ont été saturés pendant 4 heures à température ambiante dans du tampon PBS/BSA 1%. Après trois lavages en PBS/Tween 0.05%, les différents puits ont ensuite été mis en contact avec des dilutions de l'anticorps purifié Mab1F1 dans du PBS/ BSA 0.2% ( 10 ng/ml à 1 pg/ml) pendant 1 heure à température ambiante. Après trois lavages en tampon PBS/Tween 0.05%, les puits ont été incubés avec une dilution de l'anticorps anti-souris couplé à la péroxydase (dilution 1/1000 dans PBS/BSA 0.2%) (Pharmacia) pendant 1 heure à température ambiante. Après trois lavages en tampon PBS/Tween 0.05%, la réaction colorimétrique a été développée en mélangeant : 10ml d'H₂O, un comprimé OPD, 100ml de méthanol, et 25 ml d'H₂O₂. La réaction a été arrêtée avec 50ml H2SO4 4N, et la lecture spectrophotométrique a été faite à 492nm (filtre de référence 620nm).

Les résultats obtenus montrent que le peptide A est le plus réactif dans ce test et se révèle capable de reconnaître l'anticorps MablF1 dans un test ELISA. Ces résultats indiquent que l'épitope reconnu par l'anticorps Mab1F1 est situé dans le domaine définit par les acides aminés 22 à 34 de G3BP. Les mêmes expériences réalisées avec l'anticorps 1E1 ont conduit à des résultats identiques.

### (ii) Test des peptides A-F pour leur capacité à inhiber la reconnaissance de la protéine G3BP par l'anticorps Mab1F1 dans des expériences de Western-blot.

La détection des protéines par Western-blot a été réalisée selon la technique décrite dans l'exemple 2 (2.1 : Détection des protéines par Western-blot). Pour les compétitions, l'anticorps a été pré-incubé pendant deux heures à 37°C avec une dilution du peptide avant d'être mis en contact avec la membrane. Pour chaque peptide, deux concentrations ont été testées : excès molaire de 10 et de 100 par rapport à l'anticorps. La révélation a ensuite été faite comme décrit dans l'exemple 2.1.

Les résultats obtenus montrent que les peptides A et C sont capables de déplacer l'interaction entre la protéine G3BP et l'anticorps Mab1F1 (principalement avec un excès molaire 100X).

L'ensemble de ces résultats désignent le peptide A (acides aminés 22-34 de G3BP) et le peptide C (acides aminés 42-55 de G3BP) comme correspondant à des domaines de G3BP intervenant dans la reconnaissance avec l'anticorps Mab1F1.

La comparaison de séquence entre G3BP et G3BP2 forme courte a donc permis l'identification de différents peptides et la mise en évidence de leur rôle dans le contrôle de l'apoptose par G3BP, ces résultats permettent d'envisager différentes applications et notamment l'utlisation ces peptides pour de la modélisation moléculaire, afin d'obtenir de petites molécules qui miment les effets de ces peptides ou de polypeptides les incorporant. Par ailleurs, ces peptides peuvent être utilisés pour déplacer une interaction de la protéine G3BP avec un partenaire cellulaire ; ce partenaire cellulaire peut être G3BP lui-même puisqu'il a été montré que la protéine est capable de dimériser, ce partenaire peut également être un partenaire connu (RasGAP) ou un partenaire nouvellement identifié. A cet effet, ces peptides couplés à une résine peuvent être utilisés pour la recherche de partenaire cellulaire de G3BP. Enfin, un criblage de molécules d'intérêt thérapeutique peut être établi sur la base de l'inhibition d'une interaction protéine-protéine.

### Exemple 8. Construction de fragments de G3BP1 et activité apoptotique de ces fragments.

Au vu des résultats obtenus dans l'exemple 7, des plasmides permettant l'expression de fragments N-terminaux de G3BP ont été construits en vue de tester l'activité apoptotique de ces fragments. Pour cela, les fragments ont été amplifiés par PCR à partir des amorces suivantes :
A - oligonucléotide 5' dans G3BP (nucléotide 1)
   cccgtcgacatggtgatggagaagcctagtcccctg
B - oligonucléotide 5' dans G3BP (nucléotide 42)
   cccgggtcgactttgtgagacagtattacaca
C - oligonucléotide 3' dans G3BP ( nucléotide 150)
   cccgggtgcggecgcctttccatttgaatccaatcc

Les fragments (1-150 ) et (42-150) ont été amplifiés par PCR (30 cycles à 50°C), hydrolysés par les enzymes de restriction Sall et Notl, puis insérés dans le vecteur commercial pCMV / cyto ( Invitrogen, pShooter Vector manual II, version C).

Les séquences des fragments obtenus sont décrites ci-dessous : les séquences provenant du vecteur et qui seront traduites sont inscrites en majuscules, les séquences du cDNA G3BP sont indiquées en minuscules. Les régions soulignées correspondent aux séquences du vecteur codant pour le Tag myc).

Le polypeptide correspondant est présenté ci-dessous. Ce polypeptide comprend le fragment correspondant aux acides aminés 1 à 50 de la protéine G3BP dont la séquence est présentée dans la séquence SEQ ID N°1, ce fragment apparaît ci-dessous en caractère gras. Le polypeptide correspondant est présenté ci-dessous. Ce polypeptide comprend le fragment correspondant aux acides aminés 15 à 50 de la protéine G3BP dont la séquence est présentée dans la séquence SEQ ID N°1, ce fragment apparaît ci-dessous en caractère gras.

Les constructions comportant les fragments 1-150 et 42-150 de la séquence nucléotidique de G3BP ont été transfectées dans des lignées transformées NIH3T3Ras et NIH3T3Raf. Ces lignées ont été obtenues après transformation de cellules NIH3T3 par l'oncogène Ki-RasVa112, ou par la forme virale oncogénique de v-Raf.

Pour la détection de l'apoptose, 2 mg de plasmide ont été transfectés par la lipofectamine (Gibco-BRL), et l'apoptose a été mesurée 48 heures après la transfection par une analyse au FACS. Les cellules en apoptose correspondent aux cellules en phase subG1.

Dans ces conditions, la construction exprimant le fragment 1-150 de la séquence de G3BP (codant pour le fragment de G3BP comprenant les acides aminés 1 à 50) est capable d'induire l'apoptose (facteur 4) , alors que la construction exprimant le fragment 42-150 de la séquence de G3BP (codant pour le fragment de G3BP comprenant les acides aminés 15 à 50) ne présente pas cette propriété.

Ces résultats désignent le domaine correspondant aux acides aminés 1 à 14 de G3BP comme un domaine impliqué dans l'induction de l'apoptose. L'identification de ce domaine de G3BP et la mise en évidence du rôle des polypeptides comprenant le domaine correspondant aux acides aminés 1 à 14 de G3BP dans le contrôle de l'apoptose permet d'envisager l'utilisation de ces polypeptides pour la prévention, l'amélioration et/ou le traitement des pathologies impliquant une hyperprolifération cellulaire.

### Exemple 9. Construction de protéines de fusion avec des fragments de huG3BP1.

Des constructions permettant l'expression des peptides identifiés dans l'exemple 7 sont réalisées. Les séquences correspondant aux peptides sont fusionnées à la protéine GST(Glutathion S-transférase) afin de les stabiliser.

### Séquences des oligonucléotides :

Chaque oligonucleotide sens est bordé en 5' de 4 nucléotides pour reconstituer un site NcoI, et en 3' d'un codon stop (TAA) et d'un nucléotide pour former un site BamHI.
sq22s : tatgctgctgaaccaggccccagacatgctgcatagattttattaag
   sq22as : gatccttaataaaatctatgcagcatgtctggggcctggttcagcagca
   (séquence 66-105)
sq 44g 1 s : tatgggattggattcaaatggaaagccagcagatgcagtctaag
   sq44glas: gatccttagactgcatctgctggctttccatttgaatccaatccca
   (séquence 130-166)
sq44g2s : tatgggagtagatgctagtggaaagccccaggaagctgtttaag
   sq44g2as : gatccttaaacagcttcctggggctttccactagcatctactccca
   (séquence 130-166)
sq64s : tatgcagaaagaaatccacaggaaagtgatgtcacaaaacttctaag
   sq64as : gatccttagaagttttgtgacatcactttcctgtggatttctttctgca
   (séquence 172-211)
sq65s : tatgaaagtgatgtcacaaaacttcaccaactgctaag
   sq65as : gatccttagcagttggtgaagttttgtgacatcactttca
   (séquence 190-220)

Les oligonucleotides sont hybridés par paire par refroidissement de 80°C à température ambiante, phosphorylés par la T4 Polynucléotides Kinase (Boehringer), et insérés dans le vecteur « pBCcollGSTfusion » aux sites NcoI et BamHI. Ce vecteur permet l'expression dans les cellules eucaryotes d'une protéine de fusion GST à partir du promoteur SV40 (Genbank, X78316). Les plasmides sont alors transfectés dans des lignées tumorales humaines, Hela et H 1299 (décrites dans le matériel et méthodes) ; l'apoptose induite est mesurée au FACS comme décrit dans l'exemple 8.

## Revendications

1. Anticorps monoclonal capable de reconnaître un épitope compris entre les acides aminés situés aux positions 22 à 55 de la protéine G3BP et capable d'induire l'apoptose dans des cellules qui surexpriment la protéine G3BP.

2. Anticorps monoclonal selon la revendication 1, **caractérisé en ce qu'**il est capable de reconnaître un épitope constitué par les acides aminés situés aux positions 22 à 34.

3. Anticorps selon les revendications 1 ou 2, **caractérisé en ce qu'**il s'agit de l'anticorps Mab 1F1, sécrété par la lignée d'hybridome G3B 1 F1 1D1 déposée le 9 juin 1998 auprès de la C.N.C.M. sous le numéro I-2038.

4. Utilisation d'un anticorps selon l'une des revendications 1 à 3, pour l'obtention d'un médicament.

5. Utilisation d'un anticorps selon l'une des revendications 1 à 3, pour l'obtention d'un médicament destiné au traitement ou à la prévention des désordres hyperprolifératifs.

6. Composition pharmaceutique comprenant une quantité thérapeutiquement efficace des anticorps monoclonaux selon l'une des revendications 1 à 3, éventuellement mélangés à un support pharmaceutiquement acceptable, ladite quantité étant thérapeutiquement efficace pour induire l'apoptose dans des cellules tumorales.

7. Lignée d'hybridome G3B 1F1 1D1 déposée le 9 juin 1998 auprès de la C.N.C.M. sous le numéro de dépôt 1- 2038.

8. Utilisation d'un anticorps selon l'une des revendications 1 à 3, comme réactif de diagnostique.

9. Kit de diagnostic **caractérisé en ce qu'**il comprend un anticorps selon l'une des revendications 1 à 3.

## Claims

1. Monoclonal antibody capable of recognizing an epitope between the amino acids situated at positions 22 to 55 of the G3BP protein and capable of inducing apoptosis in cells that overexpress the G3BP protein.

2. Monoclonal antibody according to Claim 1, **characterized in that** it is capable of recognizing an epitope consisting of the amino acids situated at positions 22 to 34.

3. Antibody according to Claims 1 or 2, **characterized in that** it is the antibody Mab 1F1, secreted by the hybridoma line G3B 1F1 1D1 deposited on 9 June 1998 at the C.N.C.M. under the number 1-2038.

4. Use of an antibody according to one of Claims 1 to 3 for obtaining a medicine.

5. Use of an antibody according to one of Claims 1 to 3 for obtaining a medicine intended for the treatment or prevention of hyperproliferative disorders.

6. Pharmaceutical composition comprising a therapeutically effective quantity of the monoclonal antibodies according to one of Claims 1 to 3, optionally mixed with a pharmaceutically acceptable carrier, the said quantity being therapeutically effective for inducing apoptosis in tumour cells.

7. Hybridoma line G3B 1F1 1D1 deposited on 9 June 1998 at the C.N.C.M. under the deposit number 1-2038.

8. Use of an antibody according to one of Claims 1 to 3, as diagnostic reagent.

9. Diagnostic kit **characterized in that** it comprises an antibody according to one of Claims 1 to 3.

## Patentansprüche

1. Monoklonaler Antikörper, der ein Epitop zwischen den Aminosäuren an den Positionen 22 bis 55 des Proteins G3BP erkennen kann und eine Apoptose bei Zellen induzieren kann, die das Protein G3BP überexprimieren.

2. Monoklonaler Antikörper nach Anspruch 1, **dadurch gekennzeichnet, dass** er ein Epitop erkennen kann, das von den Aminosäuren an den Positionen 22 bis 34 gebildet wird.

3. Antikörper nach den Ansprüchen 1 oder 2, **dadurch gekennzeichnet, dass** es sich um den Antikörper Mab 1F1 handelt, der von der Hybridomlinie G3B 1F1 1D1 sezerniert wird, die am 9. Juni 1998 bei der C.N.C.M. unter der Nummer 1-2038 hinterlegt wurde.

4. Verwendung eines Antikörpers nach einem der Ansprüche 1 bis 3 zur Gewinnung eines Arzneimittels.

5. Verwendung eines Antikörpers nach einem der Ansprüche 1 bis 3 zur Gewinnung eines Arzneimittels, das zur Behandlung oder Vorbeugung hyperproliferativer Störungen bestimmt ist.

6. Pharmazeutische Zusammensetzung, die eine therapeutisch wirksame Menge monoklonaler Antikörper nach einem der Ansprüche 1 bis 3, gegebenenfalls im Gemisch mit einem pharmazeutisch annehmbaren Träger, umfasst, wobei die Menge therapeutisch zur Induktion einer Apoptose in Tumorzellen wirksam ist.

7. Hybridomlinie G3B 1F1 1D1, die am 9. Juni 1998 bei der C.N.C.M. unter der Hinterlegungsnummer 1-2038 hinterlegt wurde.

8. Verwendung eines Antikörpers nach einem der Ansprüche 1 bis 3 als diagnostisches Reagenz.

9. Diagnostisches Kit, **dadurch gekennzeichnet, dass** es einen Antikörper nach einem der Ansprüche 1 bis 3 umfasst.


### LISTE DE SEOUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L'INVENTION: PEPTIDE CAPABLE DE SE LIER AU DOMAINE SH3 DE LA PROTEINE GAP.
   (iii) NOMBRE DE SEQUENCES:2
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATIONS POUR LA SEQ ID NO: 1:
   (i) CARACTERISTIQUES DE LA SEQUENCE:
      (A) LONGUEUR: 466 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 1:
   Met Val Met Glu Lys Pro Ser Pro Leu Leu Val Gly Arg Glu Phe Val
   1               5                   10                  15
   Arg Gln Tyr Tyr Thr Leu Leu Asn Gln Ala Pro Asp Met Leu His Arg
               20                  25                  30
   Phe Tyr Gly Lys Asn Ser Ser Tyr Val His Gly Gly Leu Asp Ser Asn
           35                  40                  45
   Gly Lys Pro Ala Asp Ala Val Tyr Gly Gln Lys Glu Ile His Arg Lys
       50                  55                  60
   Val Met Ser Gln Asn Phe Thr Asn Cys His Thr Lys Ile Arg His Val
   65                  70                  75                  80
   Asp Ala His Ala Thr Leu Asn Asp Gly Val Val Val Gln Val Met Gly
                   85                  90                  95
   Leu Leu Ser Asn Asn Asn Gln Ala Leu Arg Arg Phe Met Gln Thr Phe
               100                 105                 110
   Val Leu Ala Pro Glu Gly Ser Val Ala Asn Lys Phe Tyr Val His Asn
               115                 120                 125
   Asp Ile Arg Tyr Gln Asp Glu Val Phe Gly Gly Phe Val Thr Glu Pro
       130                 135                 140
   Gln Glu Glu Ser Glu Glu Glu Val Glu Glu Pro Glu Glu Arg Gln Gln
   145                 150                 155                 160
   Thr Pro Glu Val Val Pro Asp Asp Ser Gly Thr Phe Tyr Asp Gln Ala
                   165                 170                 175
   Val Val Ser Asn Asp Met Glu Glu His Leu Glu Glu Pro Val Ala Glu
               180                 185                 190
   Pro Glu Pro Asp Pro Glu Pro Glu Pro Glu Gln Glu Pro Val Ser Glu
           195                 200                 205
   Ile Gln Glu Glu Lys Pro Glu Pro Val Leu Glu Glu Thr Ala Pro Glu
       210                 215                 220
   Asp Ala Gln Lys Ser Ser Ser Pro Ala Pro Ala Asp Ile Ala Gln Thr
   225                 230                 235                 240
   Val Gln Glu Asp Leu Arg Thr Phe Ser Trp Ala Ser Val Thr Ser Lys
                   245                 250                 255
   Asn Leu Pro Pro Ser Gly Ala Val Pro Val Thr Gly Ile Pro Pro His
               260                 265                 270
   Val Val Lys Val Pro Ala Ser Gln Pro Arg Pro Glu Ser Lys Pro Glu
           275                 280                 285
   Ser Gln Ile Pro Pro Gln Arg Pro Gln Arg Asp Gln Arg Val Arg Glu
       290                 295                 300
   Gln Arg Ile Asn Ile Pro Pro Gln Arg Gly Pro Arg Pro Ile Arg Glu
   305                 310                 315                 320
   Ala Gly Glu Gln Gly Asp Ile Glu Pro Arg Arg Met Val Arg His Pro
                   325                 330                 335
   Asp Ser His Gln Leu Phe Ile Gly Asn Leu Pro His Glu Val Asp Lys
               340                 345                 350
   Ser Glu Leu Lys Asp Phe Phe Gln Ser Tyr Gly Asn Val Val Glu Leu
           355                 360                 365
   Arg Ile Asn Ser Gly Gly Lys Leu Pro Asn Phe Gly Phe Val Val Phe
       370                 375                 380
   Asp Asp Ser Glu Pro Val Gln Lys Val Leu Ser Asn Arg Pro Ile Met
   385                 390                 395                 400
   Phe Arg Gly Glu Val Arg Leu Asn Val Glu Glu Lys Lys Thr Arg Ala
                   405                 410                 415
   Ala Arg Glu Gly Asp Arg Arg Asp Asn Arg Leu Arg Gly Pro Gly Gly
               420                 425                 430
   Pro Arg Gly Gly Leu Gly Gly Gly Met Arg Gly Pro Pro Arg Gly Gly
           435                 440                 445
   Met Val Gln Lys Pro Gly Phe Gly Val Gly Arg Gly Leu Ala Pro Arg
       450                 455                 460
   Gln Glx
   465
(2) INFORMATIONS POUR LA SEQ ID NO: 2 :
   (i) CARACTERISTIQUES DE LA SEQUENCE:
      (A) LONGUEUR: 2129 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 2:
   GCTTGCCTGT CAGGTCGACT CTAGAGCCCG GGTACCGAGC TCGAATTCGG CGGGGTTTGT    60
   ACTATCCTCG GTGCTGTGGT GCAGAGCTAG TTCCTCTCCA GCTCAGCCGC GTAGGTTTGG   120
   ACATATTTAC TCTTTTCCCC CCAGGTTGAA TTGACCAAAG CAATGGTGAT GGAGAAGCCT   180
   AGTCCCCTGC TGGTCGGGCG GGAATTTGTG AGACAGTATT ACACACTGCT GAACCAGGCC   240
   CCAGACATGC TGCATAGATT TTATGGAAAG AACTCTTCTT ATGTCCATGG GGGATTGGAT   300
   TCAAATGGAA AGCCAGCAGA TGCAGTCTAC GGACAGAAAG AAATCCACAG GAAAGTGATG   360
   TCACAAAACT TCACCAACTG CCACACCAAG ATTCGCCATG TTGATGCTCA TGCCACGCTA   420
   AATGATGGTG TGGTAGTCCA GGTGATGGGG CTTCTCTCTA ACAACAACCA GGCTTTGAGG   480
   AGATTCATGC AAACGTTTGT CCTTGCTCCT GAGGGGTCTG TTGCAAATAA ATTCTATGTT   540
   CACAATGATA TCTTCAGATA CCAAGATGAG GTCTTTGGTG GGTTTGTCAC TGAGCCTCAG   600
   GAGGAGTCTG AAGAAGAAGT AGAGGAACCT GAAGAAAGCA GCAAACACCT GAGGTGGTAC   660
   CTGATGATTC TGGAACTTTC TATGATCAGG CAGTTGTCAG TAATGACATG GAAGAACATT   720
   TAGAGGAGCC TGTTGCTGAA CCAGAGCCTG ATCCTGAACC AGAACCAGAA CAAGAACCTG   780
   TATCTGAAAT CCAAGAGGAA AAGCCTGAGC CAGTATTAGA AGAAACTGCC CCTGAGGATG   840
   CTCAGAAGAG TTCTTCTCCA GCACCTGCAG ACATAGCTCA GACAGTACAG GAAGACTTGA   900
   GGACATTTTC TTGGGCATCT GTGACCAGTA AGAATCTTCC ACCCAGTGGA GCTGTTCCAG   960
   TTACTGGGAT ACCACCTCAT GTTGTTAAAG TACCAGCTTC ACAGCCCCGT CCAGAGTCTA  1020
   AGCCTGAATC TCAGATTCCA CCACAAAGAC CTCAGCGGGA TCAAAGAGTG CGAGAACAAC  1080
   GAATAAATAT TCCTCCCCAA AGGGGACCCA GACCAATCCG TGAGGCTGGT GAGCAAGGTG  1140
   ACATTGAACC CCGAAGAATG GTGAGACACC CTGACAGTCA CCAACTCTTC ATTGGCAACC  1200
   TGCCTCATGA AGTGGACAAA TCAGAGCTTA AAGATTTCTT TCAAAGTTAT GGAAACGTGG  1260
   TGGAGTTGCG CATTAACAGT GGTGGGAAAT TACCCAATTT TGGTTTTGTT GTGTTTGATG   1320
   ATTCTGAGCC TGTTCAGAAA GTCCTTAGCA ACAGGCCCAT CATGTTCAGA GGTGAGGTCC   1380
   GTCTGAATGT CGAAGAGAAG AAGACTCGAG CTGCCAGGGA AGGCGACCGA CGAGATAATC   1440
   GCCTTCGGGG ACCTGGAGGC CCTCGAGGTG GGCTGGGTGG TGGAATGAGA GGCCCTCCCC   1500
   GTGGAGGCAT GGTGCAGAAA CCAGGATTTG GAGTGGGAAG GGGGCTTGCG CCACGGCAGT   1560
   AATCTTCATG GATCTTCATG CAGCCATACA AACCCTGGTT CCAACAGAAT GGTGAATTTT   1620
   CGACAGCCTT TGGTATCTTG GAGTATGACC CCAGTCTGTT ATAAACTGCT TAAGTTTGTA   1680
   TAATTTTACT TTTTTTGTGT GTTAATGGTG TGTGCTCCCT CTCCCTCTCT TCCCTTTCCT   1740
   GACCTTTAGT CTTTCACTTC CAATTTTGTG GAATGATATT TTAGGAATAA CGGACTTTTA   1800
   CCCGAATTCG TAATCATGGT CATAGCTGTT TCCGTGTGAA ATTGTTATCC GCTCACAATT   1860
   CCACACAACA TACGAGCCGG AAGCATAAAG TGTAAAGCCT GGGGTGCCTA ATGAGTGAGC   1920
   TAACTCACAT TAATTGCGTT GCGCTCACTG CCCGCTTTCC AGTCGGGAAA CCTGTCGTGC   1980
   CAGCGCATTA ATGAATCGGC CAACGCGCGG GGAGAGGCGG TTTGCGTATT GGGCGCCAGG   2040
   GTGGTTTTCT TTTCACCAGT GAGACGGGCA ACAGCTGATT GCCCTTCACC GCTGGCCCTG   2100
   AGAGAGTTGC AGCAAGCGGT CCACGCTGG                                    2129

### LISTE DE SEQUENCES

<110> Rhône-Poulenc Rorer
<120> anticorps monoclonaux dirigés contre la protéine G3BP et leurs utilisations
<130> Liste PCT
<140>
   <141>
<150> FR9807617
   <151> 1998-06-17
<160> 25
<170> PatentIn Ver. 2.1
<210> 1
   <211> 466
   <212> PRT
   <213> Homo sapiens
<400> 1
   Met Val Met Glu Lys Pro Ser Pro Leu Leu Val Gly Arg Glu Phe Val
    1               5                  10                  15
   Arg Gln Tyr Tyr Thr Leu Leu Asn Gln Ala Pro Asp Met Leu His Arg
               20                  25                  30
   Phe Tyr Gly Lys Asn Ser Ser Tyr Val His Gly Gly Leu Asp Ser Asn
           35                  40                  45
   Gly Lys Pro Ala Asp Ala Val Tyr Gly Gln Lys Glu Ile His Arg Lys
       50                  55                  60
   Val Met Ser Gln Asn Phe Thr Asn Cys His Thr Lys Ile Arg His Val
   65                  70                  75                  80
   Asp Ala His Ala Thr Leu Asn Asp Gly Val Val Val Gln Val Met Gly
                   85                  90                  95
   Leu Leu Ser Asn Asn Asn Gln Ala Leu Arg Arg Phe Met Gln Thr Phe
               100                 105                 110
   Val Leu Ala Pro Glu Gly Ser Val Ala Asn Lys Phe Tyr Val His Asn
           115                 120                 125
   Asp Ile Arg Tyr Gln Asp Glu Val Phe Gly Gly Phe Val Thr Glu Pro
       130                 135                 140
   Gln Glu Glu Ser Glu Glu Glu Val Glu Glu Pro Glu Glu Arg Gln Gln
   145                 150                 155                 160
   Thr Pro Glu Val Val Pro Asp Asp Ser Gly Thr Phe Tyr Asp Gln Ala
                   165                 170                 175
   Val Val Ser Asn Asp Met Glu Glu His Leu Glu Glu Pro Val Ala Glu
               180                 185                 190
   Pro Glu Pro Asp Pro Glu Pro Glu Pro Glu Gln Glu Pro Val Ser Glu
           195                 200                 205
   Ile Gln Glu Glu Lys Pro Glu Pro Val Leu Glu Glu Thr Ala Pro Glu
       210                 215                 220
   Asp Ala Gln Lys Ser Ser Ser Pro Ala Pro Ala Asp Ile Ala Gln Thr
   225                 230                 235                 240
   Val Gln Glu Asp Leu Arg Thr Phe Ser Trp Ala Ser Val Thr Ser Lys
                   245                 250                 255
   Asn Leu Pro Pro Ser Gly Ala Val Pro Val Thr Gly Ile Pro Pro His
               260                 265                 270
   Val Val Lys Val Pro Ala Ser Gln Pro Arg Pro Glu Ser Lys Pro Glu
           275                 280                 285
   Ser Gln Ile Pro Pro Gln Arg Pro Gln Arg Asp Gln Arg Val Arg Glu
       290                 295                 300
   Gln Arg Ile Asn Ile Pro Pro Gln Arg Gly Pro Arg Pro Ile Arg Glu
   305                 310                 315                 320
   Ala Gly Glu Gln Gly Asp Ile Glu Pro Arg Arg Met Val Arg His Pro
                   325                 330                 335
   Asp Ser His Gln Leu Phe Ile Gly Asn Leu Pro His Glu Val Asp Lys
               340                 345                 350
   Ser Glu Leu Lys Asp Phe Phe Gln Ser Tyr Gly Asn Val Val Glu Leu
           355                 360                 365
   Arg Ile Asn Ser Gly Gly Lys Leu Pro Asn Phe Gly Phe Val Val Phe
       370                 375                 380
   Asp Asp Ser Glu Pro Val Gln Lys Val Leu Ser Asn Arg Pro Ile Met
   385                 390                 395                 400
   Phe Arg Gly Glu Val Arg Leu Asn Val Glu Glu Lys Lys Thr Arg Ala
                   405                 410                 415
   Ala Arg Glu Gly Asp Arg Arg Asp Asn Arg Leu Arg Gly Pro Gly Gly
               420                 425                 430
   Pro Arg Gly Gly Leu Gly Gly Gly Met Arg Gly Pro Pro Arg Gly Gly
           435                 440                 445
   Met Val Gln Lys Pro Gly Phe Gly Val Gly Arg Gly Leu Ala Pro Arg
       450                 455                 460
   Gln Glx
   465
<210> 2
   <211> 2129
   <212> ADN
   <213> Homo sapiens
<400> 2
   gcttgcctgt caggtcgact ctagagcccg ggtaccgagc tcgaattcgg cggggtttgt 60
   actatcctcg gtgctgtggt gcagagctag ttcctctcca gctcagccgc gtaggtttgg 120
   acatatttac tcttttcccc ccaggttgaa ttgaccaaag caatggtgat ggagaagcct 180
   agtcccctgc tggtcgggcg ggaatttgtg agacagtatt acacactgct gaaccaggcc 240
   ccagacatgc tgcatagatt ttatggaaag aactcttctt atgtccatgg gggattggat 300
   tcaaatggaa agccagcaga tgcagtctac ggacagaaag aaatccacag gaaagtgatg 360
   tcacaaaact tcaccaactg ccacaccaag attcgccatg ttgatgctca tgccacgcta 420
   aatgatggtg tggtagtcca ggtgatgggg cttctctcta acaacaacca ggctttgagg 480
   agattcatgc aaacgtttgt ccttgctcct gaggggtctg ttgcaaataa attctatgtt 540
   cacaatgata tcttcagata ccaagatgag gtctttggtg ggtttgtcac tgagcctcag 600
   gaggagtctg aagaagaagt agaggaacct gaagaaagca gcaaacacct gaggtggtac 660
   ctgatgattc tggaactttc tatgatcagg cagttgtcag taatgacatg gaagaacatt 720
   tagaggagcc tgttgctgaa ccagagcctg atcctgaacc agaaccagaa caagaacctg 780
   tatctgaaat ccaagaggaa aagcctgagc cagtattaga agaaactgcc cctgaggatg 840
   ctcagaagag ttcttctcca gcacctgcag acatagctca gacagtacag gaagacttga 900
   ggacattttc ttgggcatct gtgaccagta agaatcttcc acccagtgga gctgttccag 960
   ttactgggat accacctcat gttgttaaag taccagcttc acagccccgt ccagagtcta 1020
   agcctgaatc tcagattcca ccacaaagac ctcagcggga tcaaagagtg cgagaacaac 1080
   gaataaatat tcctccccaa aggggaccca gaccaatccg tgaggctggt gagcaaggtg 1140
   acattgaacc ccgaagaatg gtgagacacc ctgacagtca ccaactcttc attggcaacc 1200
   tgcctcatga agtggacaaa tcagagctta aagatttctt tcaaagttat ggaaacgtgg 1260
   tggagttgcg cattaacagt ggtgggaaat tacccaattt tggttttgtt gtgtttgatg 1320
   attctgagcc tgttcagaaa gtccttagca acaggcccat catgttcaga ggtgaggtcc 1380
   gtctgaatgt cgaagagaag aagactcgag ctgccaggga aggcgaccga cgagataatc 1440
   gccttcgggg acctggaggc cctcgaggtg ggctgggtgg tggaatgaga ggccctcccc 1500
   gtggaggcat ggtgcagaaa ccaggatttg gagtgggaag ggggcttgcg ccacggcagt 1560
   aatcttcatg gatcttcatg cagccataca aaccctggtt ccaacagaat ggtgaatttt 1620
   cgacagcctt tggtatcttg gagtatgacc ccagtctgtt ataaactgct taagtttgta 1680
   taattttact ttttttgtgt gttaatggtg tgtgctccct ctccctctct tccctttcct 1740
   gacctttagt ctttcacttc caattttgtg gaatgatatt ttaggaataa cggactttta 1800
   cccgaattcg taatcatggt catagctgtt tccgtgtgaa attgttatcc gctcacaatt 1860
   ccacacaaca tacgagccgg aagcataaag tgtaaagcct ggggtgccta atgagtgagc 1920
   taactcacat taattgcgtt gcgctcactg cccgctttcc agtcgggaaa cctgtcgtgc 1980
   cagcgcatta atgaatcggc caacgcgcgg ggagaggcgg tttgcgtatt gggcgccagg 2040
   gtggttttct tttcaccagt gagacgggca acagctgatt gcccttcacc gctggccctg 2100
   agagagttgc agcaagcggt ccacgctgg                                   2129
<210> 3
   <211> 13
   <212> PRT
   <213> Séquence artificielle
<220>
   <223> Description de la séquence artificielle: peptide A
<400> 3
   Leu Leu Asn Gln Ala Pro Asp Met Leu His Arg Phe Tyr
   1 5 10
<210> 4
   <211> 13
   <212> PRT
   <213> Séquence artificielle
<220>
   <223> Description de la séquence artificielle: peptide B
<400> 4
   Leu Leu Asn Lys Ala Pro Glu Tyr Leu His Arg Phe Tyr
   1 5 10
<210> 5
   <211> 14
   <212> PRT
   <213> Séquence artificielle
<220>
   <223> Description de la séquence artificielle: peptide C
<400> 5
   His Gly Gly Leu Asp Ser Asn Gly Lys Pro Ala Asp Ala Val
   1 5 10
<210> 6
   <211> 14
   <212> PRT
   <213> Séquence artificielle
<220>
   <223> Description de la séquence artificielle: peptide D
<400> 6
   His Gly Gly Val Asp Ala Ser Gly Lys Pro Gln Glu Ala Val
   1 5 10
<210> 7
   <211> 14
   <212> PRT
   <213> Séquence artificielle
<220>
   <223> Description de la séquence artificielle: peptide E
<400> 7
   Leu Leu Ser Asn Asn Asn Gln Ala Leu Arg Arg Phe Met Gln
   1 5 10
<210> 8
   <211> 13
   <212> PRT
   <213> Séquence artificielle
<220>
   <223> Description de la séquence artificielle: peptide F
<400> 8
   His Asn Asp Ile Phe Arg Tyr Gln Asp Glu Val Phe Gly
   1 5 10
<210> 9
   <211> 36
   <212> ADN
   <213> Séquence artificielle
<220>
   <223> Description de la séquence artificielle: oligonucléotide 5' dans G3BP (nucléotide 1)
<400> 9
   cccgtcgaca tggtgatgga gaagcctagt cccctg 36
<210> 10
   <211> 32
   <212> ADN
   <213> Séquence artificielle
<220>
   <223> Description de la séquence artificielle: oligonucléotide 5' dans G3BP (nucléotide 42)
<400> 10
   cccgggtcga ctttgtgaga cagtattaca ca 32
<210> 11
   <211> 36
   <212> ADN
   <213> Séquence artificielle
<220>
   <223> Description de la séquence artificielle: oligonucléotide 3' dans G3BP (nucléotide 150)
<400> 11
   cccgggtgcg gccgcctttc catttgaatc caatcc 36
<210> 12
   <211> 228
   <212> ADN
   <213> Séquence artificielle
<220>
   <223> Description de la séquence artificielle: fragment 1-150
<400> 12
   atggcccagg tgcagctgca ggtcatggtg atggagaagc ctagtcccct gctggtcggg 60
   cgggaatttg tgagacagta ttacacactg ctgaaccagg ccccagacat gctgcataga 120
   ttttatggaa agaactcttc ttatgtccat gggggattgg attcaaatgg aaaggcggcc 180
   gcagaacaaa aactcatctc agaagaggat ctgaatgggg ccgcatag              228
<210> 13
   <211> 75
   <212> PRT
   <213> Séquence artificielle
<220>
   <223> Description de la séquence artificielle: traduction du fragment 1-150
<400> 13
   Met Ala Gln Val Gln Leu Gln Val Met Val Met Glu Lys Pro Ser Pro
   1               5                   10                  15
   Leu Leu Val Gly Arg Glu Phe Val Arg Gln Tyr Tyr Thr Leu Leu Asn
               20                  25                  30
   Gln Ala Pro Asp Met Leu His Arg Phe Tyr Gly Lys Asn Ser Ser Tyr
           35                  40                  45
   Val His Gly Gly Leu Asp Ser Asn Gly Lys Ala Ala Ala Glu Gln Lys
       50                  55                  60
   Leu Ile Ser Glu Glu Asp Leu Asn Gly Ala Ala
   65                  70                  75
<210> 14
   <211> 186
   <212> ADN
   <213> Séquence artificielle
<220>
   <223> Description de la séquence artificielle: fragment 42-150
<400> 14
   atggcccagg tgcagctgca ggtctttgtg agacagtatt acacactgct gaaccaggcc 60
   ccagacatgc tgcatagatt ttatggaaag aactcttctt atgtccatgg gggattggat 120
   tcaaatggaa aggcggccgc agaacaaaaa ctcatctcag aagaggatct gaatggggcc 180
   gcatag                                                            186
<210> 15
   <211> 61
   <212> PRT
   <213> Séquence artificielle
<220>
   <223> Description de la séquence artificielle: traduction du fragment 42-150
<400> 15
   Met Ala Gln Val Gln Leu Gln Val Phe Val Arg Gln Tyr Tyr Thr Leu
    1               5                  10                  15
   Leu Asn Gln Ala Pro Asp Met Leu His Arg Phe Tyr Gly Lys Asn Ser
               20                  25                  30
   Ser Tyr Val His Gly Gly Leu Asp Ser Asn Gly Lys Ala Ala Ala Glu
           35                  40                  45
   Gln Lys Leu Ile Ser Glu Glu Asp Leu Asn Gly Ala Ala
       50                  55                  60
<210> 16
   <211> 47
   <212> ADN
   <213> Séquence artificielle
<220>
   <223> Description de la séquence artificielle: polynucleotide sq22s
<400> 16
   tatgctgctg aaccaggccc cagacatgct gcatagattt tattaag 47
<210> 17
   <211> 49
   <212> ADN
   <213> Séquence artificielle
<220>
   <223> Description de la séquence artificielle: polynucleotide sq22as
<400> 17
   gatccttaat aaaatctatg cagcatgtct ggggcctggt tcagcagca 49
<210> 18
   <211> 44
   <212> ADN
   <213> Séquence artificielle
<220>
   <223> Description de la séquence artificielle: polynucleotide sq 44g1s
<400> 18
   tatgggattg gattcaaatg gaaagccagc agatgcagtc taag 44
<210> 19
   <211> 46
   <212> ADN
   <213> Séquence artificielle
<220>
   <223> Description de la séquence artificielle: polynucleotide sq44g1as
<400> 19
   gatccttaga ctgcatctgc tggctttcca tttgaatcca atccca 46
<210> 20
   <211> 44
   <212> ADN
   <213> Séquence artificielle
<220>
   <223> Description de la séquence artificielle: polynucleotide sq44g2s
<400> 20
   tatgggagta gatgctagtg gaaagcccca ggaagctgtt taag 44
<210> 21
   <211> 45
   <212> ADN
   <213> Séquence artificielle
<220>
   <223> Description de la séquence artificielle: polynucleotide sq44g2as
<400> 21
   gatccttaaa cagcttcctg gggctttcca ctagcatcta ctccc 45
<210> 22
   <211> 47
   <212> ADN
   <213> Séquence artificielle
<220>
   <223> Description de la séquence artificielle: polynucleotide sq64s
<400> 22
   tatgcagaaa gaaatccaca ggaaagtgat gtcacaaaac ttctaag 47
<210> 23
   <211> 49
   <212> ADN
   <213> Séquence artificielle
<220>
   <223> Description de la séquence artificielle: polynucleotide sq64as
<400> 23
   gatccttaga agttttgtga catcactttc ctgtggattt ctttctgca 49
<210> 24
   <211> 38
   <212> ADN
   <213> Séquence artificielle
<220>
   <223> Description de la séquence artificielle: polynucleotide sq65s
<400> 24
   tatgaaagtg atgtcacaaa acttcaccaa ctgctaag 38
<210> 25
   <211> 40
   <212> ADN
   <213> Séquence artificielle
<220>
   <223> Description de la séquence artificielle: polynucleotide sq65as
<400> 25
   gatccttagc agttggtgaa gttttgtgac atcactttca 40